# FASCICULE DE BREVET EUROPEEN

(11) **EP 3 580 195 B1**
(45) Date de publication et mention de la délivrance du brevet: **23.02.2022**
(21) Numéro de dépôt: 18702741.2
(22) Date de dépôt: 08.02.2018
(51) Int. Cl.: C07C 45/78, C07C 47/58, A23L 27/10, A23L 27/24, C12P 7/24

(54) **PROCEDE DE PURIFICATION DE LA VANILLINE NATURELLE**
VERFAHREN ZUR REINIGUNG VON NATÜRLICHEM VANILLIN
METHOD FOR THE PURIFICATION OF NATURAL VANILLIN

(30) Priorité: 08.02.2017 FR 1751021
(43) Date de publication de la demande: 18.12.2019
(73) Titulaire: Rhodia Operations, 93300 Aubervilliers (FR)
(72) Inventeur: VIBERT, Martine, 69006 Lyon (FR); ETCHEBARNE, Alain, 79500 Melle (FR)
(74) Mandataire: Valentino, Cédric
(86) Numéro de dépôt international: PCT/EP2018/053207
(87) Numéro de publication internationale: WO 2018/146210

(56) Documents cités:
- WO-A1-2014/114590
- DE-B- 1 132 113
- GB-A- 1 049 427
- US-A- 2 745 796
- US-A- 5 017 388
- US-A- 5 658 433
- AZADBAKHT M ET AL: "Preparation of lignin from wood dust as vanillin source and comparison of different extraction methods", INTERNATIONAL JOURNAL OF BIOLOGY AND BIOTECHNOLOGY, Z-A SCIENTIFIC PUBLISHER, PK, vol. 1, no. 4, 1 janvier 2004 (2004-01-01) , pages 535-537, XP009501182, ISSN: 1810-2719 cité dans la demande
- HAVKIN-FRENKEL D ET AL: "Chapter 3: Biotechnological Production of Vanillin", BIOTECHNOLOGICAL PRODUCTION OF VANILLIN, BLACKWELL PUBLISHING LTD., HOBOKEN, NJ, US, PAGE(S) 83 - 103 , 1 January 2009 (2009-01-01), XP009526527, ISBN: 978-1-4051-5649-3 Retrieved from the Internet: URL:http://doi.wiley.com/10.1002/978144430 2493.ch3 [retrieved on 2020-10-16]

## Description

### DOMAINE TECHNIQUE

La présente invention concerne un procédé de purification de la vanilline naturelle. L'invention concerne également la vanilline naturelle susceptible d'être obtenue par le procédé selon l'invention ainsi qu'un dispositif pour produire de la vanilline naturelle.

### ART ANTERIEUR

La vanilline peut être obtenue par différentes méthodes connues de l'homme de l'art, et notamment par les deux voies suivantes :
- Une voie dite naturelle basée sur un procédé biotechnologique comprenant notamment la culture d'un microorganisme apte à permettre la biotransformation d'un substrat de fermentation en vanilline. Il est notamment connu de la demande EP0885968 un tel procédé dans lequel le substrat de fermentation est l'acide férulique. Le brevet US 5017388 décrit un procédé dans lequel le substrat de fermentation est l'eugénol et/ou l'isoeugénol. Ces procédés aboutissent à la préparation d'une vanilline dite vanilline naturelle.
- Une voie dite synthétique comprenant des réactions chimiques classiques à partir du gaïacol ne faisant pas intervenir de microorganisme. Ce procédé aboutit à la préparation d'une vanilline dite vanilline synthétique.

Enfin la vanilline peut également être préparée selon une voie qualifiée de naturelle dans laquelle la vanilline est issue de lignine, on peut citer en particulier les documents US 2745796, DE1132113 et l'article intitulé « Preparation of lignin from wood dust as vanillin source and comparison of different extraction method » by Azadbakht et al in International Journal of Biology and Biotechnology, 2004, vol 1, No 4, pp 535-537.

Actuellement la vanilline naturelle peut être purifiée selon le procédé décrit dans la demande EP 2791098 qui comprend une étape d'extraction liquide/liquide d'impuretés ayant un pKa plus élevé que celui de la vanilline. Le rendement de ce procédé est bon, en général supérieur à 80%, toutefois afin d'obtenir des qualités améliorées quant à la couleur de la vanilline plusieurs étapes de purification additionnelles sont nécessaires faisant ainsi chuter le rendement global de ce procédé.

La demande internationale WO 2014/114590 décrit également un procédé de purification de vanilline naturelle. Ce procédé consiste à évaporer la vanilline naturelle, cette évaporation pouvant être effectuée par distillation ou par évaporation sous vide de vanilline fondue. Ce procédé est susceptible de produire de la vanilline naturelle très pure, avec un bon rendement, avec un dispositif simple à mettre en œuvre et fonctionnant de façon continue pour être compatible avec les procédés industriels. Cependant, un tel procédé pourrait être difficile à mettre en pratique en raison du nombre et de la dimension des équipements nécessaires.

C'est pourquoi il serait avantageux de disposer d'un procédé plus simple par rapport à ceux proposés dans l'art antérieur.

Un des objectifs de la présente invention est de fournir un procédé ayant un rendement global amélioré tout en obtenant une vanilline présentant une coloration nettement améliorée, en particulier une coloration en solution éthanolique à 10% inférieure ou égale à 100 Hazen, préférentiellement inférieure ou égale à 50 Hazen, et plus préférentiellement inférieure ou égale à 20 Hazen, et encore plus préférentiellement inférieure ou égale à 10 Hazen.

### RESUME DE L'INVENTION

L'objet de la présente invention concerne un procédé de purification de la vanilline naturelle, issue d'un procédé de fermentation d'acide férulique, ledit procédé de purification comprenant au moins une étape (b) de stripping d'un flux liquide F2 comprenant de la vanilline naturelle avec un gaz d'entraînement G1 et/ou d'un liquide vaporisé L1 dans lequel la concentration massique de la vanilline naturelle dans le flux liquide F2 est supérieure ou égale à 10% et caractérisé en ce que l'étape (b) est réalisée sous atmosphère inerte.

### DESCRIPTION DETAILLEE DE L'INVENTION

Dans le cadre de la présente invention, et sauf indication contraire, l'expression « compris entre ...et... » inclut les bornes.

L' objet de la présente invention concerne un procédé de purification de la vanilline naturelle, issue d'un procédé de fermentation d'acide férulique, ledit procédé de purification comprenant au moins une étape (b) de stripping d'un flux liquide F2 comprenant de la vanilline naturelle avec un gaz d'entraînement G1 et/ou d'un liquide vaporisé L1 dans lequel la concentration massique de la vanilline naturelle dans le flux liquide F2 est supérieure ou égale à 10% et caractérisé en ce que l'étape (b) est réalisée sous atmosphère inerte.

Le procédé selon l'invention permet avantageusement d'obtenir de la vanilline naturelle avec un titre élevé, à partir d'un flux liquide F2 comprenant de la vanilline naturelle.

Dans la présente invention, l'expression « vanilline naturelle » peut notamment désigner une substance aromatisante naturelle selon l'article 9.2.c) du règlement CE1334/2008, c'est-à-dire une substance aromatisante obtenue par des procédés physiques, enzymatiques ou microbiologiques à partir de matières d'origine végétale, animale ou microbiologique prises en l'état ou après leur transformation pour la consommation humaine par un ou plusieurs des procédés traditionnels de préparation des denrées alimentaires. Une substance aromatisante naturelle correspond à une substance qui est naturellement présente et a été identifiée dans la nature. Les définitions données par les réglementations en vigueur dans d'autres pays ou zones du monde peuvent également s'appliquer. D'autre part, « vanilline naturelle » peut par ailleurs désigner de la vanilline extraite de gousses de vanille.

Avantageusement, le flux liquide F2 peut être issu d'un procédé de production de vanilline naturelle. Dans ce flux F2, la vanilline naturelle se présente sous forme de vanilline non salifiée. De préférence, dans le flux liquide F2 de vanilline naturelle, la concentration massique de la vanilline est supérieure ou égale à 10%, préférentiellement supérieure ou égale à 30%, plus préférentiellement supérieure ou égale à 50% et encore plus préférentiellement supérieure ou égale à 70% par rapport à la masse totale dudit flux liquide.

De préférence, un procédé de production de vanilline naturelle désigne ici un procédé biotechnologique comprenant la culture d'un microorganisme apte à permettre la biotransformation d'un substrat de fermentation en vanilline. Il s'agit ici d'un procédé de fermentation d'acide férulique, tel que celui décrit dans la demande de brevet EP 0885968.

Outre la vanilline, le flux F2 peut contenir des impuretés, en particuliers des impuretés formées lors de la production par conversion enzymatique ou par fermentation, typiquement l'alcool vanillique, l'acide vanillique, des dimères et des trimères de la vanilline (c'est-à-dire des composés présentant un squelette ayant respectivement deux ou trois groupements phényles, les dimères étant avantageusement choisis parmi les diphényleméthanes). Lorsqu'il s'agit d'une fermentation d'acide férulique, les impuretés typiques peuvent en outre être choisies parmi l'acide férulique, le gaïacol et des dérivés du gaïacol. Enfin, le flux F2 peut contenir des traces de stabilisant.

En outre, le flux F2 peut comprendre un solvant ou un mélange de plusieurs solvants, tels qu'un solvant alimentaire (par exemple les solvants identifiés par le programme FEMA GRAS^{™}) ou de l'eau. Toutefois, la concentration massique de solvant dans le flux liquide F2 est préférentiellement inférieure ou égale à 90%, plus préférentiellement inférieure ou égale à 70%, plus préférentiellement inférieure ou égale à 50% et encore plus préférentiellement inférieure ou égale à 30%, par rapport à la masse totale dudit flux liquide.

Le procédé selon l'invention peut être mis en œuvre selon un fonctionnement en mode continu ou selon un fonctionnement en mode discontinu.

Le stripping est une étape d'entraînement via un gaz d'entrainement ou un liquide vaporisé. Des impuretés sont en effet entraînées par le gaz ou liquide vaporisé de manière à améliorer la qualité de la vanilline.

En particulier dans le cadre de la présente invention, l'étape de stripping permet avantageusement d'éliminer certaines impuretés contenues dans le flux de vanilline naturelle.

Une telle étape de stripping peut être réalisée dans des conditions douces, notamment par injection d'un fluide gazeux ou d'un liquide et/ou mise sous vide de l'enceinte où est opéré le procédé selon l'invention.

Dans le cadre de la présente invention, le gaz d'entraînement G1 ou le liquide vaporisé L1 est choisi dans le groupe constitué par l'eau, la vapeur d'eau, les acétates d'alkyle, les alcools, les gaz inertes choisis parmi N₂, CO₂, He, Ar, air appauvri et leurs mélanges. Préférentiellement, le gaz d'entraînement G1 ou le liquide vaporisé L1 est l'eau ou la vapeur d'eau. L'utilisation d'un mélange de plusieurs gaz d'entraînement G1 et/ou de plusieurs liquides vaporisés L1 est envisageable.

L'étape (b) peut être réalisée à une température supérieure ou égale à 20°C, de préférence supérieure ou égale à 30°C, de manière plus préférentielle supérieure ou égale à 40°C, et encore plus préférentielle supérieure ou égale à 50°C. L'étape (b) peut être réalisée à une température inférieure ou égale à 140°C, de préférence inférieure ou égale à 120°C, de manière plus préférentielle inférieure ou égale à 100°C, et encore plus préférentielle à 95°C. Selon un mode de réalisation de la présente invention, l'étape (b) est réalisée sous vide décroissant allant de 400 mbar à 25 mbar.

Selon un premier mode de réalisation, l'étape de stripping est réalisée avec un gaz d'entraînement G1, de préférence avec de la vapeur d'eau. Ce mode de réalisation peut être mis en œuvre en continu dans une colonne de stripping : typiquement, le flux liquide F2 est introduit dans la colonne de stripping par le haut, tandis qu'un gaz d'entrainement G1 est introduit par le bas. Lors de leur contact, le gaz d'entrainement se charge en impuretés contenues dans le flux liquide, et est extrait par le haut de la colonne, tandis que le flux liquide purifié est récupéré en pied de colonne.

Selon un second mode de réalisation, l'étape de stripping est réalisée avec un liquide vaporisé L1, de préférence avec de l'eau. Ce mode de réalisation peut être mis en œuvre en continu ou en discontinu dans un réacteur : typiquement, le flux liquide F2 et le liquide L1 sont mélangés dans le réacteur. Puis, par diminution de la pression et/ou augmentation de la température, le liquide L1 se vaporise. Le liquide vaporisé L1 est alors extrait du réacteur, en entrainant avec lui des impuretés contenues dans le flux liquide. Ledit flux liquide ainsi purifié peut alors être récupéré dans le réacteur.

Préférentiellement, l'étape (b) de stripping est réalisée sous N₂.

Selon un mode de réalisation préféré, le procédé de purification de la vanilline naturelle selon l'invention peut comprendre en outre une étape préliminaire (a) de préparation dudit flux liquide F2 de vanilline naturelle en évaporant, éventuellement en présence d'eau, le solvant SI d'un flux F1 provenant de la production de vanilline naturelle.

En effet, lors de la production de vanilline naturelle, il est typique de récupérer un flux liquide comprenant de la vanilline naturelle, des impuretés et une grande quantité de solvant, typiquement un solvant alimentaire tel que par exemple l'acétate d'éthyle. Ce flux est typiquement dénommé « solution de vanilline brute ». Le procédé selon l'invention peut comprendre une étape de mise à disposition d'un flux liquide F1 issu d'un procédé de production de vanilline naturelle comprenant de la vanilline naturelle dans un solvant S1. Parmi les solvants S1, on peut par exemple citer les solvants organiques autorisés par la réglementation tels que la MEK (méthyl-éthylcétone), les alcools (éthanol, butanol...), les acétates d'alkyle (acétate d'éthyle, acétate de propyle, acétate d'isopropyle...), la MIBK (méthyl-isobutylcétone) et le cyclohexane. Le solvant SI peut également être de l'eau. Le solvant SI peut également être un mélange de solvants organiques, notamment un mélange des solvants organiques cités ci-dessus ou un mélange d'eau et d'un solvant organique.

Selon un mode de réalisation, le flux liquide F1 peut comprendre en outre un stabilisant du milieu de fermentation. Les agents bactériostatiques (ou biocides) pouvant être employés comme stabilisants sont bien connus de l'homme du métier, par exemple l'acide sorbique, l'acide benzoïque, l'acide acétique et leurs sels. Le stabilisant de la vanilline naturelle dans le flux liquide F1 est généralement présent en quantité inférieure ou égale à 20% en poids, préférentiellement inférieur ou égal à 10% en poids. Le flux liquide F2 peut en conséquence contenir des traces desdits stabilisants. Toutefois, le flux F1 peut également être dépourvu de stabilisant, en particulier lorsque la stabilisation du milieu de fermentation a été effectuée d'une autre manière. Par exemple, le flux liquide F1 peut avoir été stabilisé par traitement thermique. Ce traitement thermique permet de stopper l'action des microorganismes. La température du traitement thermique est en général supérieure ou égale à 35°C et en général inférieure ou égale à 110°C, préférentiellement entre 50°C et 110°C.

Selon un mode de réalisation particulier de la présente invention, le procédé comprend en outre une étape préliminaire, avant l'étape (a) ou avant l'étape (b), de lavage du flux issu du procédé de production de vanilline naturelle. Ce lavage peut être réalisé en utilisant une solution aqueuse afin d'éliminer les impuretés acides. En général ce lavage peut être réalisé avec une solution basique, de préférence une solution de soude.

De préférence, dans le flux F1, la concentration massique de la vanilline est comprise de 0,5% à 60%, plus préférentiellement de 5% à 40%, et encore plus préférentiellement de 10% à 35% par rapport à la masse totale dudit flux.

L'étape optionnelle (a) selon la présente invention consiste à éliminer le solvant SI pour obtenir un flux F2 de vanilline dans laquelle la concentration massique de la vanilline est supérieure ou égale à 10%, préférentiellement supérieure ou égale à 30%, plus préférentiellement supérieure ou égale à 50% et encore plus préférentiellement supérieure ou égale à 70% par rapport à la masse totale dudit flux. La teneur résiduelle en solvant SI est préférentiellement inférieure ou égale à 90%, plus préférentiellement inférieure ou égale à 70%, plus préférentiellement inférieure ou égale à 50% et encore plus préférentiellement inférieure ou égale à 30% par rapport à la masse totale dudit flux liquide.

Conformément au procédé selon l'invention, l'étape d'évaporation (a) peut éventuellement être réalisée en présence d'eau, laquelle est ajoutée au flux F1 avant et/ou pendant la mise en œuvre de ladite étape d'évaporation.

Selon un mode de réalisation préféré, dans le cadre de l'étape (a), le solvant SI de la vanilline brute est éliminé par évaporation, par exemple par distillation ou au moyen d'un évaporateur, en présence d'eau. L'eau et le solvant SI peuvent forment un mélange azéotrope. Dans le cas d'une évaporation par distillation, le solvant SI peut être distillé à pression atmosphérique ou sous vide ou encore à pression atmosphérique puis sous vide. L'eau peut être ajoutée en une ou plusieurs fois au flux F1. Il est préférable d'utiliser de l'eau alimentaire (par exemple de l'eau de ville). On peut également utiliser une eau alimentaire recyclée, provenant du procédé selon la présente invention.

De préférence, ladite étape d'évaporation a) est effectuée à une température comprise entre 60 et 140°C et très préférentiellement entre 80 et 100°C.

Après l'étape (b), le procédé de purification de la vanilline naturelle selon l'invention peut comprendre en outre une étape (c) d'élimination des composés moins volatils que la vanilline. Cette étape peut avantageusement être réalisée dans un évaporateur à film sous vide, ou dans un évaporateur à film raclé.

La température du fluide caloporteur à laquelle est réalisée l'étape (c) est généralement supérieure ou égale à 130°C, préférentiellement supérieure ou égale à 145°C, et inférieure ou égale à 230°C, préférentiellement inférieure ou égale à 180°C. La pression à laquelle est réalisée l'étape (c) est généralement supérieure ou égale à 0,4 mbar, préférentiellement supérieure ou égale à 1 mbar, et généralement inférieure ou égale à 75 mbar, préférentiellement inférieure à 8 mbar, et plus préférentiellement inférieure ou égale à 4 mbar.

Cette étape (c) d'évaporation peut être facilitée par l'emploi d'adjuvants techniques, par exemple par l'ajout d'un fluidifiant. Ainsi, selon un mode de réalisation particulier de la présente invention, un fluidifiant autorisé par les règlementations sur les produits alimentaires, par exemple du polyéthylène glycol, peut être ajouté à la vanilline naturelle entre l'étape (b) et l'étape (c), ou pendant l'étape (c).

A l'issue de ladite étape (c), on obtient avantageusement un condensat de vanilline naturelle qui n'est pas coloré. Ledit condensat de vanilline naturelle est préférentiellement de couleur inférieure ou égale à 400 Hazen, de préférence inférieure ou égale à 200 Hazen, plus préférentiellement inférieure ou égale à 100 Hazen, plus préférentiellement inférieure ou égale à 50 Hazen et encore plus préférentiellement inférieure ou égale à 20 Hazen (en solution éthanolique à 10% en poids).

Le procédé selon la présente invention peut comprendre en outre une étape (d) de mise en forme de la vanilline naturelle, de préférence par cristallisation, de manière plus préférentielle par cristallisation dans un solvant identifié par le programme FEMA GRAS^{™}. Dans un mode de réalisation préféré, la vanilline naturelle est cristallisée ou recristallisée dans l'eau ou dans un mélange alcool/eau. La vanilline ainsi obtenue est sous forme de cristaux blancs. Selon un mode de réalisation préféré de l'invention le solvant utilisé est compatible avec les réglementations permettant la fabrication de produits utilisables dans l'industrie alimentaire.

La vanilline naturelle susceptible d'être obtenue selon le procédé de l'invention est caractérisée en ce qu'elle se présente sous la forme d'un solide dont la couleur, en solution éthanolique à 10% en poids, est inférieure ou égale à 200 Hazen, de préférence inférieure ou égale 100 Hazen, préférentiellement inférieure ou égale à 50 Hazen, plus préférentiellement inférieure ou égale à 20 Hazen (en solution éthanolique à 10% en poids).

Son titre en vanilline est avantageusement supérieur ou égal à 95%, préférentiellement supérieur ou égal à 99%, plus préférentiellement supérieur ou égal à 99,5%.

La vanilline naturelle susceptible d'être obtenue selon le procédé de l'invention est caractérisée en ce qu'elle présente un profil organoleptique conforme.

La vanilline naturelle susceptible d'être obtenue selon le procédé de l'invention présente des impuretés spécifiques. Les impuretés contenues dans la vanilline naturelle obtenue selon le procédé de l'invention sont liées au procédé de préparation de ladite vanilline naturelle. Ainsi les impuretés présentent dans un moût de fermentation de vanilline naturelle issue d'acide férulique sont différentes de celles obtenues dans un moût de fermentation d'eugénol ou d'isoeugénol. Par ailleurs les méthodes de purifications permettent d'éliminer certaines impuretés et dans des quantités variables, ainsi à l'issue de l'étape de purification, la vanilline naturelle présentera des impuretés qui sont propres à son procédé de préparation et propres à son procédé de purification.

Les inventeurs ont découvert que de façon tout à fait surprenante la vanilline naturelle issue d'un procédé de fermentation et purifiée conformément à l'invention présente un profil organoleptique conforme, en particulier en terme d'aspect visuel, de texture, de goût, d'odeur.

Comparé aux procédés de l'art antérieur, le procédé de purification selon la présente invention permet de purifier la vanilline naturelle avec un rendement supérieur ou égal à 70%, préférentiellement supérieur ou égale à 80%, plus préférentiellement supérieur ou égal à 90%. La vanilline naturelle obtenue présente avantageusement un titre très élevé en vanilline, elle est de couleur blanche et présente un profil organoleptique conforme, sans fausses notes, tel que montré en exemple. L'évaluation de la conformité peut notamment être réalisée par un panel entraîné en test triangulaire par rapport à la référence. La Norme ISO 4120:2004(f) définit le nombre minimal de réponses correctes pour établir une différence significative.

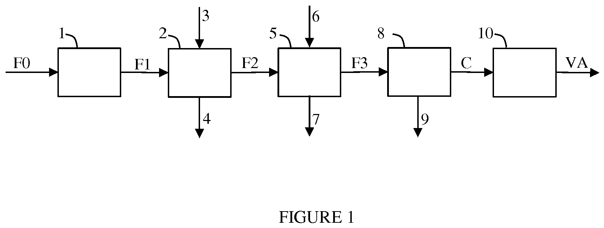

Un mode de réalisation particulier, mais non limitatif, du procédé selon l'invention est représenté sur la FIGURE 1.

Selon ce mode de réalisation, une solution de vanilline brute (F0) est obtenue par un procédé de production de vanilline naturelle. Cette solution comprend de la vanilline naturelle dans un solvant SI et des impuretés typiques du procédé de production. Cette solution F0 est lavée à l'aide d'une solution de soude dans un dispositif de lavage (1). Le flux (F1) obtenu comprend de la vanilline naturelle dans le solvant SI et une teneur réduite en impuretés acides.

Le flux (F1) est introduit dans un dispositif de distillation (2) de manière à évaporer le solvant S1. De l'eau est éventuellement introduite en (3). Tandis que le solvant SI et l'eau sont évacués en (4), on récupère un flux (F2) comprenant avantageusement plus de 70% de vanilline naturelle.

Le flux (F2) est introduit dans un dispositif de stripping (5). Les impuretés sont éliminées par un gaz d'entraînement G1 et/ou un liquide vaporisé L1 introduit en (6) et évacué en (7).

Le flux de vanilline naturelle purifié (F3) est alors traité dans un évaporateur à film sous vide ou à film raclé (8). Les composés lourds sont séparés en (9) et on obtient un condensat de vanilline naturelle (C) de haute pureté et non coloré.

Ledit condensat (C) peut être cristallisé en (10), pour obtenir la vanilline naturelle (VA) sous forme de cristaux blancs.

La présente invention va maintenant être illustrée par des exemples qui ne présentent pas un caractère limitatif à l'invention.

### EXEMPLES (de référence)

### Exemple 1 :

Le procédé illustré sur le FIGURE 1 a été mis en œuvre à partir d'un flux issu d'un procédé de production de vanilline naturelle en solution. Ce flux présentait la composition suivante :

| Composé | Pourcentage massique |
|---|---|
| Vanilline | 17,3% |
| Alcool vanillique | 2,3% |
| Acide benzoïque | 1,5% |
| Gaïacol | 0,1% |

Ce flux a été soumis à une première étape de lavage avec une solution aqueuse de soude. Le flux obtenu à l'issue de cette étape comprend les composés suivants :

| Composé | Pourcentage massique |
|---|---|
| Vanilline | 16,9% |
| Alcool vanillique | 1,9% |
| Acide benzoïque | 0,3% |
| Gaïacol | 0,1% |

Une étape (a) de distillation en présence d'eau a été réalisée à une température de 80°C sous une pression de 100 mbar. Le flux à la sortie comprenait les composés suivants :

| Composé | Pourcentage massique |
|---|---|
| Vanilline | 78,1% |
| Alcool vanillique | 10,0% |
| Acide benzoïque | 1,5% |

Une étape (b) de stripping a été réalisée sur le flux obtenu à l'issue de l'étape (a), à une température de 90°C, en utilisant comme liquide d'entraînement de l'eau qui est vaporisée dans les conditions opératoires : de l'eau liquide a été ajoutée à pression atmosphérique, puis la pression a été abaissée jusqu'à 25 mbar. Le flux à la sortie comprenait les composés suivants :

| Composé | Pourcentage massique |
|---|---|
| Vanilline | 74,5% |
| Alcool vanillique | 8,5% |
| Acide benzoïque | 1,2% |

Une étape (c) dans un évaporateur à film raclé, a ensuite été réalisée. Le flux à la sortie comprenait les composés suivants :

| Composé | Pourcentage massique |
|---|---|
| Vanilline | 92,4% |
| Alcool vanillique | 6,6% |

Enfin une étape (d) de cristallisation a été réalisée sur le produit obtenu en sortie de l'étape (c). La vanilline obtenue à l'issue de ce procédé de purification présente un titre de 99,6% et est obtenue avec un rendement de 87%.

La vanilline ainsi purifiée présente une colorimétrie de 18 Hazen.

Une analyse sensorielle selon un essai triangulaire a été réalisée en conformité avec la norme ISO 4120/2004 portant sur les essais triangulaires. L'analyse sensorielle est réalisée sur un panel composé de 7 à 11 personnes. Suite à la réalisation de cette analyse sensorielle, il a été conclu que les qualités organoleptiques de la vanilline obtenue sont conformes à la référence, la référence étant ici de la vanilline commerciale Rhovanil^{®} Natural CW.

### Exemple 2 :

On procède comme dans l'exemple 1 sauf que le flux initial présentait la composition suivante :

| Composé | Pourcentage massique |
|---|---|
| Vanilline | 20,3% |
| Alcool vanillique | 2,3% |
| Acide benzoïque | 0% |
| Gaïacol | 0,3% |
| Autres impuretés | 0,7% |

L'étape de lavage n'a pas été faite. Après distillation, le flux à la sortie comprenait les composés suivants :

| Composé | Pourcentage massique |
|---|---|
| Vanilline | 85,6% |
| Alcool vanillique | 9,7% |
| Gaïacol | 1,3% |
| Autres impuretés | 3,4% |

Le stripping a été réalisée avec de la vapeur d'eau. Pour cela, 600g du flux à traité ont été placés dans un réacteur de IL muni d'un condenseur. On a injecté par plongeant de la vapeur d'eau basse pression à 104-108°C pendant 3 h. Le flux à la sortie comprenait les composés suivants :

| Composé | Pourcentage massique |
|---|---|
| Vanilline | 96,6% |
| Alcool vanillique | 9,5% |
| Autres impuretés | 3,9% |

Après passage dans un évaporateur à film raclé, le flux à la sortie comprenait les composés suivants :

| Composé | Pourcentage massique |
|---|---|
| Vanilline | 90,2% |
| Alcool vanillique | 9,2% |

Après cristallisation, la vanilline présente un titre de 99,5%.

## Revendications

1. Procédé de purification de la vanilline naturelle, issue d'un procédé de fermentation d'acide férulique, ledit procédé de purification comprenant au moins une étape (b) de stripping d'un flux liquide F2 comprenant de la vanilline naturelle avec un gaz d'entraînement G1 et/ou d'un liquide vaporisé L1 dans lequel la concentration massique de la vanilline naturelle dans le flux liquide F2 est supérieure ou égale à 10% et **caractérisé en ce que** l'étape (b) est réalisée sous atmosphère inerte.

2. Procédé de purification de la vanilline naturelle selon la revendication 1 dans lequel la concentration massique de la vanilline naturelle dans le flux liquide F2 est supérieure ou égale à 30%.

3. Procédé de purification de la vanilline naturelle selon la revendication 1 dans lequel la concentration massique de la vanilline naturelle dans le flux liquide F2 est supérieure ou égale à 50%.

4. Procédé de purification de la vanilline naturelle selon la revendication 1 dans lequel la concentration massique de la vanilline naturelle dans le flux liquide F2 est supérieure ou égale à 70%.

5. Procédé de purification de la vanilline naturelle selon l'une quelconque des revendications 1 à 4 comprenant en outre une étape préliminaire (a) de préparation dudit flux liquide F2 de vanilline naturelle en évaporant, éventuellement en présence d'eau, le solvant SI d'un flux F1 provenant de la production de vanilline naturelle.

6. Procédé de purification de la vanilline naturelle selon l'une quelconque des revendications 1 à 5 comprenant en outre après l'étape (b), une étape (c) d'élimination des composés moins volatils que la vanilline, préférentiellement dans un évaporateur à film sous vide ou dans un évaporateur à film raclé.

7. Procédé de purification de la vanilline naturelle selon l'une quelconque des revendications 1 à 6 dans lequel le gaz d'entraînement G1 ou le liquide vaporisé L1 est choisi dans le groupe constitué par l'eau, la vapeur d'eau, les acétates d'alkyle, les alcools, les gaz inertes choisis parmi N₂, CO₂, He, Ar, air appauvri et leurs mélanges.

8. Procédé de purification de la vanilline naturelle selon l'une quelconque des revendications 1 à 7 dans lequel le gaz d'entraînement G1 ou le liquide vaporisé L1 est l'eau ou la vapeur d'eau.

9. Procédé de purification de la vanilline naturelle selon l'une quelconque des revendications 1 à 8 dans lequel l'étape (b) est réalisée à une température supérieure ou égale à 20°C, de préférence supérieure ou égale à 30°C, de manière plus préférentielle supérieure ou égale à 40°C, et encore plus préférentielle supérieure ou égale à 50°C, et à une température inférieure ou égale à 140°C, de préférence inférieure ou égale à 120°C, de manière plus préférentielle inférieure ou égale à 100°C, et encore plus préférentielle à 95°C.

10. Procédé de purification de la vanilline naturelle selon l'une quelconque des revendications 1 à 9, **caractérisée en ce que** la vanilline naturelle obtenu à l'étape (b) ou (c) est de couleur inférieure ou égale à 400 Hazen, de préférence inférieure ou égale à 200 Hazen, plus préférentiellement inférieure ou égale à 100 Hazen, plus préférentiellement inférieure ou égale à 50 Hazen et encore plus préférentiellement inférieure ou égale à 20 Hazen (en solution éthanolique à 10% en poids).

11. Procédé selon l'une quelconque des revendications 1 à 10 comprenant en outre une étape (d) de mise en forme de la vanilline naturelle, de préférence par cristallisation.

## Patentansprüche

1. Verfahren zur Aufreinigung von natürlichem Vanillin, welches aus einem Verfahren zur Fermentation von Ferulasäure stammt, wobei das Aufreinigungsverfahren zumindest einen Schritt (b) umfasst, in welchem ein flüssiger Stoffstrom F2, der natürliches Vanillin umfasst, einer austreibenden Behandlung mit einem Trägergas G1 und/oder einer verdampften Flüssigkeit L1 unterzogen wird, wobei die massenbezogene Konzentration des natürlichen Vanillins in dem flüssigen Stoffstrom F2 mindestens 10 % beträgt, wobei es **dadurch gekennzeichnet ist, dass** der Schritt (b) unter Inertgasatmosphäre durchgeführt wird.

2. Verfahren zur Aufreinigung von natürlichem Vanillin nach Anspruch 1, wobei die massenbezogene Konzentration des natürlichen Vanillins in dem flüssigen Stoffstrom F2 mindestens 30 % beträgt.

3. Verfahren zur Aufreinigung von natürlichem Vanillin nach Anspruch 1, wobei die massenbezogene Konzentration des natürlichen Vanillins in dem flüssigen Stoffstrom F2 mindestens 50 % beträgt.

4. Verfahren zur Aufreinigung von natürlichem Vanillin nach Anspruch 1, wobei die massenbezogene Konzentration des natürlichen Vanillins in dem flüssigen Stoffstrom F2 mindestens 70 % beträgt.

5. Verfahren zur Aufreinigung von natürlichem Vanillin nach einem beliebigen der Ansprüche 1 bis 4, wobei es darüber hinaus einen vorgeschalteten Schritt (a) umfasst, in welchem der flüssige Stoffstrom F2 aus natürlichem Vanillin bereitgestellt wird, indem das Lösungsmittel S1 eines Stoffstroms F1, welcher aus der Produktion von natürlichem Vanillin stammt, verdampft wird, möglicherweise in Gegenwart von Wasser.

6. Verfahren zur Aufreinigung von natürlichem Vanillin nach einem beliebigen der Ansprüche 1 bis 5, welches darüber hinaus nach dem Schritt (b) einen Schritt (c) umfasst, in welchem Verbindungen, die weniger flüchtig als Vanillin sind, entfernt werden, vorzugsweise in einem Dünnschichtverdampfer unter Vakuum und in einem Dünnschichtverdampfer mit Rührblatt.

7. Verfahren zur Aufreinigung von natürlichem Vanillin nach einem beliebigen der Ansprüche 1 bis 6, wobei das Trägergas G1 oder die verdampfte Flüssigkeit L1 aus der Gruppe ausgewählt sind, welche aus Wasser, Wasserdampf, Alkylacetaten, Alkoholen, Inertgasen besteht, wobei letztere aus N₂, CO₂, He, Ar, abgereicherter Luft und deren Mischungen besteht.

8. Verfahren zur Aufreinigung von natürlichem Vanillin nach einem beliebigen der Ansprüche 1 bis 7, wobei es sich bei dem Trägergas G1 oder der verdampften Flüssigkeit L1 um Wasser oder Wasserdampf handelt.

9. Verfahren zur Aufreinigung von natürlichem Vanillin nach einem beliebigen der Ansprüche 1 bis 8, wobei der Schritt (b) bei einer Temperatur von mindestens 20 °C, vorzugsweise von mindestens 30 °C, stärker bevorzugt von mindestens 40 °C, und noch stärker bevorzugt von mindestens 50 °C, sowie bei einer Temperatur von höchstens 140 °C, vorzugsweise von höchstens 120 °C, stärker bevorzugt von höchstens 100 °C, und noch stärker bevorzugt bei 95 °C durchgeführt wird.

10. Verfahren zur Aufreinigung von natürlichem Vanillin nach einem beliebigen der Ansprüche 1 bis 9, **dadurch gekennzeichnet, dass** das natürliche Vanillin, welches in Schritt (b) oder (c) erhalten wird, eine Farbzahl hat, die höchstens 400 Hazen, vorzugsweise höchstens 200 Hazen, stärker bevorzugt höchstens 100 Hazen, stärker bevorzugt höchstens 50 Hazen und noch stärker bevorzugt höchstens 20 Hazen beträgt (in ethanolischer Lösung zu 10 Gewichts-%).

11. Verfahren nach einem beliebigen der Ansprüche 1 bis 10, wobei es darüber hinaus einen Schritt (d) umfasst, in welchem das natürliche Vanillin, vorzugsweise durch Kristallisation, eine Formgebung erfährt.

## Claims

1. Process for the purification of natural vanillin, resulting from a process for the fermentation of ferulic acid, said purification process comprising at least one step (b) of stripping a liquid stream F2 comprising natural vanillin with an entraining gas G1 and/or a vaporized liquid L1, in which the concentration by weight of natural vanillin in the liquid stream F2 is greater than or equal to 10% and **characterized in that** step (b) is carried out under an inert atmosphere.

2. Process for the purification of natural vanillin according to Claim 1, in which the concentration by weight of natural vanillin in the liquid stream F2 is greater than or equal to 30%.

3. Process for the purification of natural vanillin according to Claim 1, in which the concentration by weight of natural vanillin in the liquid stream F2 is greater than or equal to 50%.

4. Process for the purification of natural vanillin according to Claim 1, in which the concentration by weight of natural vanillin in the liquid stream F2 is greater than or equal to 70%.

5. Process for the purification of natural vanillin according to any one of Claims 1 to 4, additionally comprising a preliminary step (a) of preparation of said liquid stream F2 of natural vanillin by evaporating, optionally in the presence of water, the solvent S1 of a stream F1 originating from the production of natural vanillin.

6. Process for the purification of natural vanillin according to any one of Claims 1 to 5, additionally comprising, after step (b), a step (c) of removal of the less volatile compounds than vanillin, preferably in a vacuum film evaporator or in a thin film evaporator.

7. Process for the purification of natural vanillin according to any one of Claims 1 to 6, in which the entraining gas G1 or the vaporized liquid L1 is chosen from the group consisting of water, steam, alkyl acetates, alcohols, inert gases chosen from N₂, CO₂, He, Ar, depleted air and their mixtures.

8. Process for the purification of natural vanillin according to any one of Claims 1 to 7, in which the entraining gas G1 or the vaporized liquid L1 is water or steam.

9. Process for the purification of natural vanillin according to any one of Claims 1 to 8, in which step (b) is carried out at a temperature of greater than or equal to 20°C, preferably of greater than or equal to 30°C, more preferably of greater than or equal to 40°C and more preferably still of greater than or equal to 50°C, and at a temperature of less than or equal to 140°C, preferably of less than or equal to 120°C, more preferably of less than or equal to 100°C and more preferable still at 95°C.

10. Process for the purification of natural vanillin according to any one of Claims 1 to 9, **characterized in that** the natural vanillin obtained in step (b) or (c) has a colour of less than or equal to 400 Hazen, preferably of less than or equal to 200 Hazen, more preferably of less than or equal to 100 Hazen, more preferably of less than or equal to 50 Hazen and more preferably still of less than or equal to 20 Hazen (in 10% by weight ethanolic solution).

11. Process according to any one of Claims 1 to 10, additionally comprising a step (d) of forming natural vanillin, preferably by crystallization.
